# EUROPÄISCHE PATENTSCHRIFT

(11) **EP 1 093 369 B1**
(45) Veröffentlichungstag und Bekanntmachung des Hinweises auf die Patenterteilung: **27.11.2002**
(21) Anmeldenummer: 99934560.6
(22) Anmeldetag: 02.07.1999
(51) Int. Cl.: A61K 31/425, A61K 31/135, A61P 25/24

(54) **MITTEL MIT ANTIDEPRESSIVER WIRKUNG, ENTHALTEND PRAMIPEXOL UND SERTRALIN**
AGENT WITH AN ANTIDEPRESSIVE EFFECT COMPRISING PRAMIPEXOLE AND SERTRALINE
AGENT A EFFET ANTIDEPRESSIF CONTENANT DU PRAMIPEXOLE ET DE LA SERTRALINE

(30) Priorität: 07.07.1998 DE 19830201
(43) Veröffentlichungstag der Anmeldung: 25.04.2001
(73) Patentinhaber: Boehringer Ingelheim Pharma KG, 55218 Ingelheim am Rhein (DE)
(72) Erfinder: MAJ, Jerzy, PL-31-228 Kraków (PL)
(74) Vertreter: Laudien, Dieter, Dr.
(86) Internationale Anmeldenummer: EP9904595
(87) Internationale Veröffentlichungsnummer: WO00002542

(56) Entgegenhaltungen:
- WO-A-94/13287
- WO-A-98/24446
- WO-A-99/36064
- MAJ, JERZY ET AL: "The behavioral effects of pramipexole, a novel dopamine recepto agonist" EUR. J. PHARMACOL. (1997), 324(1), 31-37, XP000884558

## Beschreibung

Die vorliegende Erfindung betrifft ein Mittel mit antidepressiver Wirkung enthaltend 2-Amino-4,5,6,7-tetrahydro-6-propylamino-benzothizol, sein (+) oder (-) Enantiomer, deren pharmakologisch verträgliche Säureadditionssalze und Sertralin.

### Stand der Technik

Pramipexol - das (-)-2-Amino-6-n-propylamino-4,5,6,7-tetrahydrobenzo-thiazol - ist ein Dopamin - D₃/D₂ Agonist, dessen Synthese in dem europäischen Patent 186 087 und dem US-Patent 4,886,812 beschrieben ist. Pramipexol ist in erster Linie zur Behandlung der Schizophrenie und insbesondere zur Behandlung des Parkinsons bekannt. Aus der deutschen Patentanmeldung DE 38 43 227 ist offenbart, daß Pramipexol den Prolactinserumspiegel senkt, weiterhin ist aus der deutschen Patentanmeldung DE 39 33 738 bekannt, Pramipexol zur Senkung hoher TSH-Spiegel einzusetzen. Die transdermale Applikation ist in dem US-Patent 5,112,842 offenbart, und die WO Patentanmeldung PCT/EP93/03389 beschreibt die Verwendung von Pramipexol als Antidepressivum.

Einzelheiten zur Herstellung der Titelverbindung können der EP-A 85 116 016 entnommen werden, auf die dort zitierte Literatur wird hiermit ausdrücklich verwiesen.

### Beschreibung der Erfindung

Überraschenderweise wurde nun gefunden, daß Pramipexol, sein (+) oder (-) Enantiomer oder deren pharmakologisch verträgliche Säureadditionssalze in Kombination mit Sertralin eine deutlich höhere antidepressive Wirkung entfaltet als es die beiden Einzelkomponenten für sich bewirken können. Besonders hervorzuheben ist der sofortige Wirkungseintritt der Wirkstoffkombination.

Die verbesserte Wirkung von Pramipexol durch gleichzeitige Gabe eines weiteren Antidepressivums wurde in Untersuchungen an Ratten, denen Pramipexol und Sertralin gegeben wurde, im sogenannten "forced swimming test" gefunden. Einzelheiten zu dieser Untersuchungsmethode finden sich z.B. bei Willner, Psychopharmacology 83, 1-16 (1984) oder Borsini und Meli, Psychopharmacology 94, 147-160 (1988).

Für die erfindungsgemäße Kombination aus Pramipexol und Sertralin (1S-cis)-4-(3,4-Dichlorphenyl)-1,2,3,4-tetrahydro-N-methyl-1-naphthalenamine, bzw. deren Säureadditionssalze, wurde der Test folgendermaßen durchgeführt. Die Tiere wurden in verschiedene Gruppen aufgeteilt und je eine der Gruppen erhielt entweder eine Kochsalzlösung, eine therapeutisch wirksame Menge Pramipexol, eine therapeutische Menge Sertralin oder eine Kombination der beiden Antidepressiva in jeweils der gleichen therapeutischen Menge wie die Tiere, die ausschließlich einen der beiden Wirkstoffe erhielten.

Besonders bevorzugt ist die Kombination von 2-Amino-4,5,6,7-tetrahydro-6-propylamino-benzothiazol, sein (+) oder (-) - Enantiomer, deren verträgliche Säureadditionssalze und (1S-cis)-4-(3,4-Dichlorphenyl)-1,2,3,4-tetrahydro-N-methyl-1-naphthalenamine (Sertralin) sowie dessen Säureadditionssalze - ganz besonders bevorzugt in die Kombination von Pramipexol und Sertralin jeweils in Form ihrer Hydrochloride.

Der Begriff Kombination versteht sich erfindungsgemäß als Wirkstoffkombination der beiden Wirkstoffe in einer Formulierung und auch als Kombination im Sinne von Einzelformulierungen der Wirkstoffe, die in einer zeitlich zusammenhängenden therapeutischen Maßnahme angewendet werden.
Oral applizierbare pharmazeutische Formulierungen für Pramipexol sind aus dem Stand der Technik bekannt und z.B. auf dem deutschen oder US-amerikanischen Markt erhältlich.

Die einzelnen Wirkstoffe können auch als Kitt im Sinne einer Packungskombination der einzelnen Arzneimittel, wie auch getrennt abgepackt sein.

Die Kombination aus Pramipexol und einem weiteren Antidepressivum kann analog den üblichen galenischen Zubereitungen formuliert werden, in der Regel zusammen mit einem pharmazeutischen Träger. D.h. eine effektive Dosis der Einzelkomponenten und gegebenenfalls ein pharmazeutischer Träger werden als Tablette, Dragee, Kapsel, Oblate, Pulver, Lösung, Suspension, Emulsion, Sirup, Suppositorium usw. formuliert. Für Pramipexol liegt die pharmazeutisch wirksame Dosis pro Patient zwischen 0,01 und 10 mg, bevorzugt zwischen 0.08 und 5 mg.

Die therapeutisch wirksame Dosis von Sertralin in der Kombination ist 50 mg.

In der erfindungsgemäßen Kombination kann die empfohlene Dosierung in Einzelfällen auch unterhalb der bisherigen empfohlenen Einzeldosierung des Monopräparates liegen.

### Beschreibung der Versuche

Pramipexol wurde in Dosierungen von 0.1 und 0.3 mg/kg eingesetzt. Zusätzlich wurden Untersuchungen mit 0.05 mg/kg Pramipexol durchgeführt. Sertralin wurde wie in den Tabellen aufgeführt, in Dosierungen von 5 und 10 mg/kg eingesetzt. Die Versuche wurden an Ratten (male Wistar, 250-270 g) bei RT unter Einhaltung eines natürlichen Tag-Nacht-Rhythmus durchgeführt. Pramipexol (HCI) wurde in einer physiologischen Kochsalzlösung und Sertralin (HCl) in destilliertem Wasser gelöst, beide Substanzen wurden in einem Volumen von 2 ml/kg injiziert.

### Forced swimming Test bei Ratten

Die gesamte Immobilitätszeit wurde nach Porsolt et al. (1978) innerhalb einer fünfminütigen Beobachtungsperiode bestimmt. Pramipexol (0.05, 0.1 und 0.3 mg/kg) und Sertralin (5 oder 10 mg/kg) wurde dreimal im Abstand von 24,5 und 1 Stunde vor dem Test gegeben.

In gesonderten Gruppen wurde Pramipexol in oben genannter Dosierung zusammen mit Sertralin (5 oder 10 mg/kg) ebenfalls dreimal wie oben beschrieben injiziert. Jede Gruppe bestand aus 10 Ratten.

### Ergebnisse

Pramipexol - 0.1 mg/kg - verändert nicht die Immobilitätszeit im forced swimming test, während höhere Dosen (0.3 mg) eine signifikante Verringerung der Immobilitätszeit bewirkt.

Eine Dosierung von 5 mg/kg Sertralin allein reduzieren ebenfalls nicht die Immobilitätszeit. Aber die gemeinsame Applikation von 5 mg/kg Sertralin und 0.1 mg/kg Pramipexol verringern merklich die Immobilitätszeit. Wesentlich ausgeprägter tritt dieser Effekt bei höheren Dosierungen von Sertralin auf.

Sertralin allein in einer Dosis von 10 mg/kg war im forced swimming Test inaktiv, aber in Kombination mit Pramipexol gegeben (0.1, 0.3 mg/kg). Dieser Effekt verstärkt sich bei höheren Dosierungen von Pramipexol. Pramipexol in der Dosierung von 0.05 mg/kg zeigt keinen Einfluß auf die Immobilitätszeit, aber in Kombination mit Sertralin zeigt sich eine Reduzierung der Immobilitätszeit.

Diese Ergebnisse belegen die unerwartete synergistische Wirkung von Pramipexol in Kombination mit Sertralin als Antidepressivum.

**Tabelle 1.**

| Wirkung von Pramipexol (0.1 und 0.3 mg/kg) allein oder in Kombination mit Sertralin (5 mg/kg) auf die Immobilitätszeit im forced swimming test bei Ratten. | | |
|---|---|---|
| Verbindungen (mg/kg) | Immobilitätszeit (en) | |
| | mean ± SEM | P |
| 1. Vehicle | 239.9 ± 3.1 | ― |
| 2. Sertraline 5 | 257.0 ± 7.0 | ns vs 1 |
| 3. Pramipexole 0.1 | 223.4 ± 6.2 | ns vs 1 |
| 4. Pramipexole 0.3 | 171.5 ± 9.2 | <0.001 vs 1 |
| 5. Sertraline 5 + Pramipexole 0.1 | 96.1 ± 10.3 | <0.001 vs 3 |
| 6. Sertraline 5 + Pramipexole 0.3 | 18.1 ± 3.5 | <0.001 vs 4 |
| Pramipexol (0.1 oder 0.3 mg/kg s.c.) und Sertralin (5 mg/kg i.p.) werden 3mal (24,5 und 1 Stunde) vor dem Test verabreicht. | | |

**Tabelle 2.**

| Wirkung von Pramipexol (0.1 und 0.3 mg/kg) allein oder in Kombination mit Sertralin (10 mg/kg) auf die Immobilitätszeit im forced swimming test bei Ratten. | | |
|---|---|---|
| Verbindungen (mg/kg) | Immobilitätszeit (en) | |
| | mean ± SEM | P |
| 1. Vehicle | 237.9 ± 2.7 | ― |
| 2. Sertraline 10 | 223.6 ± 9.9 | ns vs 1 |
| 3. Pramipexole 0.1 | 212.5 ± 6.9 | ns vs 1 |
| 4. Pramipexole 0.3 | 142.9 ± 7.9 | <0.001 vs 1 |
| 5. Sertraline 10 + Pramipexole 0.1 | 133.3 ± 6.9 | <0.001 vs 3 |
| 6. Sertraline 10 + Pramipexole 0.3 | 11.8 ± 2.3 | <0.001 vs 4 |
| Pramipexol (0.1 oder 0.3 mg/kg s.c.) und Sertralin (10 mg/kg i.p.) werden 3mal (24,5 und 1 Stunde) vor dem Test verabreicht. | | |

**Tabelle 3.**

| Wirkung von Pramipexol (0.05 mg/kg) allein oder in Kombination mit Sertralin (5 und 10 mg/kg) auf die Immobilitätszeit im forced swimming test bei Ratten. | | |
|---|---|---|
| Verbindungen (mg/kg) | Immobilitätszeit (en) | |
| | mean ± SEM | P |
| 1. Vehide | 235.3 ± 4.8 | ― |
| 2. Pramipexole 0.05 | 245.5 ± 7.8 | ns vs 1 |
| 3. Sertraline 5 | 247.5 ± 3.0 | ns vs 1 |
| 4. Sertraline 10 | 223.7 ± 2.8 | ns vs 1 |
| 5. Sertraline 5 + Pramipexole 0.05 | 187.7 ± 11.2 | <0.001 vs 2 |
| 6. Sertraline 10 + Pramipexole 0.05 | 163.9 ± 10.0 | <0.001 vs 2 |
| Pramipexol (0.05 mg/kg s.c.) und Sertralin (5 und 10 mg/kg i.p.) werden 3mal (24,5 und 1 Stunde) vor dem Test verabreicht. | | |

## Patentansprüche

1. Mittel zur Behandlung von Depressionen enthaltend 2-Amino-4,5,6,7-tetrahydro-6-propylamino-benzothiazol, eines seiner Enatiomeren oder Säureadditionssalze davon in Kombination mit Sertralin oder einem seiner pharmakologisch verträglichen Salze.

2. Mittel nach Anspruch 1, **dadurch gekennzeichnet, daß** es das (+) - Enatiomer des 2-Amino-4,5,6,7-tetrahydro-6-propylamino-benzothiazol oder eines seiner Säureadditionssalze enthält.

3. Mittel nach Anspruch 1, **dadurch gekennzeichnet, daß** es das (-) - Enantiomer des 2-Amino-4,5,6,7-tetrahydro-6-propylamino-benzothiazol oder eines seiner pharmakologisch verträglichen Säuresalze enthält.

4. Mittel nach einem der Ansprüche 1 bis 3, **dadurch gekennzeichnet, daß** es das 2-Amino-4,5,6,7-tetrahydro-6-propylamino-benzothiazol-dihydrochlorid insbesondere das 2-Amino-4,5,6,7-tetrahydro-6-propylamino-benzothiazoldihydrochloridmonohydrat enthält.

5. Mittel gemäß Anspruch 1, **dadurch gekennzeichnet, daß** es 0,05 - 10 mg 2-Amino-4,5,6,7-tetrahydro-6-propylamino-benzothiazol, eines seiner Enatiomeren oder eines seiner Säureadditionssalze enthält.

6. Mittel gemäß Anspruch 1, **dadurch gekennzeichnet, daß** es 0,05 - 10 mg Pramipexol oder Pramipexol-dihydrochlorid-monohydrat enthält.

7. Mittel gemäß Anspruch 1, **dadurch gekennzeichnet, daß** es 0,088 - 1,5 mg 2-Amino-4,5,6,7-tetrahydro-6-propylamino-benzothiazol, eines seiner Enatiomeren oder eines seiner Säureadditionssalze enthält.

8. Mittel gemäß Anspruch 1, **dadurch gekennzeichnet, daß** es 0,088 - 1,5 mg Pramipexol oder Pramipexol-dihydrochloridmonohydrat enthält.

9. Mittel nach Anspruch 8, **dadurch gekennzeichnet, daß** es zwischen 0,088 und 1,1 mg Pramipexol oder zwischen 0,125 und 1,5mg Pramipexoldihydrochloridmonohydratmonohydrat enthält.

10. Mittel nach einem der Ansprüche 1 bis 9, **dadurch gekennzeichnet, daß** es zwischen 25 und 200 mg Sertralin enthält.

11. Mittel nach Anspruch 10, **dadurch gekennzeichnet, daß** es 50 mg Sertralin enthält.

12. Verwendung eines Mittels gemäß einem der vorhergehenden Ansprüche zur Herstellung eines Medikaments zur Behandlung von Depressionen oder depressiven Zuständen.

13. Verwendung eines Mittels gemäß einem der vorhergehenden Ansprüche zur Herstellung eines Arzneimittels zur Behandlung von Depressionen.

14. Verwendung eines Mittels gemäß einem der vorhergehenden Ansprüche zur Herstellung einer Medikation zur Behandlung von Depressionen, **dadurch gekennzeichnet, daß** die Wirkstoffe des Mittels als Einzelverbindungen zeitlich nacheinander einzunehmen sind.

## Claims

1. Agent for treating depression, containing 2-amino-4,5,6,7-tetrahydro-6-propylamino-benzothiazole, one of the enantiomers thereof or one of the acid addition salts thereof in conjunction with sertraline or one of the pharmacologically acceptable salts thereof .

2. Agent according to claim 1, **characterised in that** it contains the (+)-enantiomer of 2-amino-4,5,6,7-tetrahydro-6-propylamino-benzothiazole or one of the acid addition salts thereof.

3. Agent according to claim 1, **characterised in that** it contains the (-)-enantiomer of 2-amino-4,5,6,7-tetrahydro-6-propylamino-benzothiazole or one of the pharmacologically acceptable acid addition salts thereof.

4. Agent according to one of claims 1 to 3, **characterised in that** it contains 2-amino-4,5,6,7-tetrahydro-6-propylamino-benzothiazole-dihydrochloride, particularly 2-amino-4,5,6,7-tetrahydro-6-propylamino-benzothiazoledihydrochloride monohydrate.

5. Agent according to claim 1, **characterised in that** it contains 0.05-10 mg of 2-amino-4,5,6,7-tetrahydro-6-propylamino-benzothiazole, one of the enantiomers or one of the acid addition salts thereof.

6. Agent according to claim 1, **characterised in that** it contains 0.05-10 mg of pramipexole or pramipexole dihydrochloride monohydrate.

7. Agent according to claim 1, **characterised in that** it contains 0.088 - 1.5 mg of 2-amino-4,5,6,7-tetrahydro-6-propylamino-benzothiazole, one of the enantiomers or one of the acid addition salts thereof.

8. Agent according to claim 1, **characterised in that** it contains 0.088 - 1.5 mg of pramipexole or pramipexole dihydrochloride monohydrate.

9. Agent according to claim 8, **characterised in that** it contains between 0.088 and 1.1 mg of pramipexole or between 0.125 and 1.5 mg of pramipexole dihydrochloride monohydrate monohydrate.

10. Agent according to one of claims 1 to 9, **characterised in that** it contains between 25 and 200 mg of sertraline.

11. Agent according to claim 10, **characterised in that** it contains 50 mg of sertraline.

12. Use of an agent according to one of the preceding claims for preparing a medicament for treating depression or depressive states.

13. Use of an agent according to one of the preceding claims for preparing a pharmaceutical composition for treating depression.

14. Use of an agent according to one of the preceding claims for preparing a medicament for treating depression, **characterised in that** the active substances of the agent are to be taken as individual substances one after the other over time.

## Revendications

1. Agent pour le traitement de la dépression, contenant le 2-amino-4,5,6,7-tétrahydro-6-propylaminobenzothiazole, un de ses énantiomères ou sels d'addition d'acide en combinaison avec la sertraline ou un de ses sels pharmacologiquement compatibles.

2. Agent selon la revendication 1, **caractérisé en ce qu'**il contient l'énantiomère (+) du 2-amino-4,5,6,7-tétrahydro-6-propylaminobenzothiazole ou un de ses sels d'addition d'acide.

3. Agent selon la revendication 1, **caractérisé en ce qu'**il contient l'énantiomèr (-) du 2-amino-4,5,6,7-tétrahydro-6-propylaminobenzothiazole ou un de ses sels d'addition d'acide pharmacologiquement compatibles.

4. Agent selon l'une quelconque des revendications 1 à 3, **caractérisé en ce qu'**il contient le dichlorhydrate du 2-amino-4,5,6,7-tétrahydro-6-propylaminobenzothiazole, en particulier le dichlorhydrate monohydraté du 2-amino-4,5,6,7-tétrahydro-6-propylaminobenzothiazole.

5. Agent selon la revendication 1, **caractérisé en ce qu'**il contient 0,05 - 10 mg du 2-amino-4,5,6,7-tétrahydro-6-propylaminobenzothiazole, d'un de ses énantiomères ou d'un de ses sels d'addition d'acide.

6. Agent selon la revendication 1, **caractérisé en ce qu'**il contient 0,05 - 10 mg de pramipexol ou du dichlorhydrate monohydraté du pramipexol.

7. Agent selon la revendication 1, **caractérisé en ce qu'**il contient 0,088 - 1,5 mg du 2-amino-4,5,6,7-tétrahydro-6-propylaminobenzothiazole, d'un de ses énantiomères ou d'un de ses sels d'addition d'acide.

8. Agent selon la revendication 1, **caractérisé en ce qu'**il contient 0,088 - 1,5 mg de pramipexol ou du dichlorhydrate monohydraté du pramipexol.

9. Agent selon la revendication 8, **caractérisé en ce qu'**il contient de 0,088 à 1,1 mg de pramipexol ou de 0,125 à 1,5 mg du dichlorhydrate monohydraté du pramipexol.

10. Agent selon l'une quelconque des revendications 1 à 9, **caractérisé en ce qu'**il contient de 25 à 200 mg de sertraline.

11. Agent selon la revendication 10, **caractérisé en ce qu**'il contient 50 mg de sertraline.

12. Utilisation d'un agent selon l'une quelconque des revendications précédentes, pour la préparation d'un médicament pour le traitement de la dépression ou d'états dépressifs.

13. Utilisation d'un agent selon l'une quelconque des revendications précédentes, pour la préparation d'un agent pharmaceutique pour le traitement de la dépression.

14. Utilisation d'un agent selon l'une quelconque des revendications précédentes, pour la préparation d'une médication pour le traitement de la dépression, **caractérisé en ce que** les agents actifs sont à prendre l'un après l'autre, en tant que composés individuels.
